# EUROPEAN PATENT APPLICATION

(11) **EP 1 188 840 A2**
(43) Date of publication of application: **20.03.2002**
(21) Application number: 01306262.5
(22) Date of filing: 20.07.2001
(51) Int. Cl.: C12Q 1/68, B01L 3/00

(54) **Chemical reaction method and apparatus**

(30) Priority: 26.07.2000 US 626419
(71) Applicant: Agilent Technologies, Inc., Palo Alto, CA 94305 (US)
(72) Inventor: Caren, Michael P., Palo Alto, California 94303 (US); Mitchell, J. Robert, San Francisco, California 94131 (US)
(74) Representative: Jehan, Robert

(57) **Abstract**

Methods and apparatus are disclosed for carrying out multiple chemical reactions such as polynucleotide hybridization reactions. A substrate (12) is brought into proximity with a reaction medium that comprises reagents for carrying out the reactions. The substrate comprises a plurality of heat generating elements (14). The heat generating elements (14) are selectively activated and deactivated to control temperature of the medium adjacent predetermined areas on the substrate thereby controlling the chemical reactions adjacent the predetermined areas. The substrate can further comprise a plurality of temperature sensing elements and the method comprises selectively activating the temperature sensing elements to monitor the temperature adjacent the predetermined areas on the substrate (12). The substrate (12) can further comprise a plurality of electrodes in which case the method comprises selectively activating the electrodes to control movement of the reagents relative to the substrate.

## Description

This invention relates to methods and apparatus for carrying out chemical reactions such as hybridization reactions involving biopolymers. The preferred embodiments can be used in the field of bioscience in which arrays of polynucleotide probes, fabricated or deposited on a surface, are used to identify polynucleotide sequences such as DNA sequences in cell matter. They have a wide range of application for conducting cell study, for diagnosing disease, identifying gene expression, monitoring drug response, determining of viral load, identifying genetic polymorphisms, and the like.

Significant morbidity and mortality are associated with infectious diseases and genetically inherited disorders. More rapid and accurate diagnostic methods are required for better monitoring and treatment of these conditions. Molecular methods using DNA probes, nucleic acid hybridization and in vitro amplification techniques are promising methods offering advantages to conventional methods used for patient diagnoses. For example, the ability to clone and synthesize nucleotide sequences has led to the development of a number of techniques for disease diagnosis and genetic analysis. Genetic analysis, including correlation of genotypes and phenotypes, contributes to the information necessary to reveal the changes in genes that confer disease. New methods of diagnosis of diseases, such as AIDS, cancer, sickle cell anemia, cystic fibrosis, diabetes, muscular dystrophy, and the like, rely on the detection of mutations present in certain nucleotide sequences.

Nucleic acid hybridization has been employed for investigating the identity and establishing the presence of nucleic acids. Hybridization is based on complementary base pairing. When complementary single stranded nucleic acids are incubated together, the complementary base sequences pair to form double-stranded hybrid molecules. The ability of single stranded deoxyribonucleic acid (ssDNA) or ribonucleic acid (RNA) to form a hydrogen bonded structure with a complementary nucleic acid sequence has been employed as an analytical tool in molecular biology research. The availability of radioactive nucleoside triphosphates of high specific activity and the development of methods for their incorporation into DNA and RNA has made it possible to identify, isolate, and characterize various nucleic acid sequences of biological interest. Nucleic acid hybridization has great potential in diagnosing disease states associated with unique nucleic acid sequences. These unique nucleic acid sequences may result from genetic or environmental change in DNA by insertions, deletions, point mutations, or by acquiring foreign DNA or RNA by means of infection by bacteria, molds, fungi, and viruses.

The application of nucleic acid hybridization as a diagnostic tool in clinical medicine is limited due to the cost and effort associated with the development of sufficiently sensitive and specific methods for detecting potentially low concentrations of disease-related DNA or RNA present in the complex mixture of nucleic acid sequences found in patient samples.

One method for detecting nucleic acids is to employ nucleic acid probes that have sequences complementary to sequences in the target nucleic acid. A nucleic acid probe may be, or may be capable of being, labeled with a reporter group or may be, or may be capable of becoming, bound to a support. Detection of signal depends upon the nature of the label or reporter group. Usually, the probe is comprised of natural nucleotides such as ribonucleotides and deoxyribonucleotides and their derivatives although unnatural nucleotide mimetics such as 2'-modified nucleosides, peptide nucleic acids and oligomeric nucleoside phosphonates are also used. Commonly, binding of the probes to the target is detected by means of a label incorporated into the probe. Alternatively, the probe may be unlabeled and the target nucleic acid labeled. Binding can be detected by separating the bound probe or target from the free probe or target and detecting the label. In one approach, a sandwich is formed comprised of one probe, which may be labeled, the target and a probe that is or can become bound to a surface. Alternatively, binding can be detected by a change in the signal-producing properties of the label upon binding, such as a change in the emission efficiency of a fluorescent or chemiluminescent label. This permits detection to be carried out without a separation step. Finally, binding can be detected by labeling the target, allowing the target to hybridize to a surface-bound probe, washing away the unbound target and detecting the labeled target that remains.

The targets or probes are usually immobilized on a solid support having a surface area of a few square centimeters to millimeters. The solid support is typically a glass or fused silica slide, which has been treated to facilitate attachment of either the targets or probes. The mobile phase containing reactants that react with the attached targets or probes is placed on the support, covered with another slide, and placed in an environmentally controlled chamber such as an incubator. Normally, the reactants in the mobile phase diffuse through the liquid to the interface where the complementary probes or targets are immobilized, and a reaction, such as hybridization reaction, then occurs. Preferably, the mobile phase reactants are labeled with a detectable tag, such as a fluorescent tag, so that the hybrid can be identified. The hybridization reaction typically takes place over a time period that can be many hours and even up to a day or more.

Direct detection of labeled target hybridized to surface-bound probes is particularly advantageous if the surface contains a mosaic of different probes that are individually localized to discrete, known areas of the surface. Such ordered arrays containing a large number of oligonucleotide probes have been developed as tools for high throughput analyses of genotype and gene expression. Oligonucleotides synthesized on a solid support recognize uniquely complementary nucleic acids by hybridization, and arrays can be designed to define specific target sequences, analyze gene expression patterns or identify specific allelic variations.

In one approach, cell matter is lysed, to release its DNA as fragments, which are then separated out by electrophoresis or other means, and then tagged with a fluorescent or other label. The resulting DNA mix is exposed to an array of oligonucleotide probes, whereupon selective attachment to matching probe sites takes place. The array is then washed and imaged so as to reveal for analysis and interpretation the sites where attachment occurred.

DNA arrays have become an effective means for researchers to look at thousands of genes in a single experiment. Typical hybridization stations consist of a consumable array flow cell and an automated fluidic station. The procedure consists of loading the sample into the consumable, hybridizing the sample at an appropriate temperature with a mixing means, removing the sample and finally washing the flow cell. After hybridization, the consumable is scanned for the results of the assay and then the consumable is discarded. Although this architecture has proved to be adequate, it addresses all of its functionality through indirect means. First, it accomplishes its DNA movement by moving bulk fluids and relying on diffusion for specific interaction. Second, temperature control is provided by placing the array in an oven and relying on convection to the array and then conduction through the array to the hybridization surface. This is a dynamically slow control means, which can also be inaccurate depending upon the thermal layout of the system.

An integrated nucleic acid diagnostic device is discussed in US-5,922,591. US-5,624,815 discloses method and apparatus for the analysis of biological material. Total internal reflection optical switches employing thermal activation is discussed in US-5,699,462. Apparatus and a method for mixing of a film of fluid via nucleation of bubbles within the film is disclosed in United States patent application no. 09/137,963 filed August 21, 1998 (Schembri).

The present invention seeks to provide improved hybridisation reactions.

According to an aspect of the present invention, there is provided a method of carrying out hybridisation reactions as specified in claim 1.

According to another aspect of the present invention, there is provided apparatus for conducting hybridisation reactions as specified in claim 10.

The present invention also provides a method of carrying out chemical reactions, including the steps of bringing into proximity with a reaction medium a substrate comprising a plurality of heat generating elements, said reaction medium comprising reagents for carrying out said reactions, and selectively activating and deactivating said heat generating elements to control temperature of said medium adjacent predetermined areas on said substrate thereby controlling said reactions adjacent said predetermined areas.

One embodiment provides a method of carrying out chemical reactions. A substrate is brought into proximity with a reaction medium that comprises reagents for carrying out the reactions. The substrate comprises a plurality of heat generating elements. The heat generating elements are selectively activated and deactivated to control the temperature of the medium adjacent predetermined areas on the substrate thereby controlling the chemical reactions adjacent the predetermined areas. The substrate can further comprise a plurality of temperature sensing elements and the method can further comprise selectively activating the temperature sensing elements to monitor the temperature adjacent the predetermined areas on the substrate. The substrate can further comprise a plurality of electrodes and the method can further comprise selectively activating the electrodes to control movement of the reagents relative to the substrate.

Another embodiment provides a method of carrying out reactions involving biopolymers. A reaction medium is brought into proximity with a substrate comprising a plurality of electrodes, a plurality of heat generating elements and a plurality of temperature sensing elements. The reaction medium comprises reagents for carrying out the reactions. The electrodes are selectively activated to control movement of the reagents to and from predetermined areas adjacent to the substrate. The heat generating elements are selectively activated and deactivated to control temperature of the medium adjacent predetermined areas on the substrate thereby controlling reaction of biopolymers adjacent the predetermined areas. The temperature sensing elements are selectively activated to monitor the temperature adjacent the predetermined areas on the substrate. In one embodiment the surface of the substrate comprises biopolymers attached thereto in a predetermined pattern.

Another embodiment provides apparatus for conducting reactions involving biopolymers. The apparatus comprises a substrate, a plurality of discrete heat generating elements associated with the substrate and arranged in a predetermined pattern, and a plurality of discrete temperature sensing elements associated with the substrate and arranged in a predetermined pattern. In one embodiment the apparatus can comprise a plurality of electrodes associated with the substrate and arranged in a predetermined pattern.

Another embodiment provides a method of carrying out polynucleotide hybridization reactions. A reaction medium is brought into proximity with a substrate, which comprises a plurality of resistors and an array of polynucleotides attached thereto. The reaction medium comprises reagents for carrying out the polynucleotide hybridization reactions. The resistors are selectively activated and deactivated to control temperature of the medium adjacent predetermined areas on the substrate. In this way hybridization of polynucleotides is controlled at the predetermined areas and hybridized polynucleotides are formed on the surface with unhybridized polynucleotides remaining in solution. In one embodiment the substrate comprises a plurality of electrodes and the method comprises selectively activating the electrodes to control movement of the reagents to and from predetermined areas on the substrate. In another embodiment the substrate comprises a plurality of thermistors and the method comprises selectively activating the temperature sensing elements to monitor temperature adjacent the predetermined areas on the substrate.

Embodiments of the present invention are described below, by way of example only, with reference to the accompanying drawings, in which:
Fig. 1 is a schematic diagram of one embodiment of apparatus;
Fig. 2 is a schematic diagram of a substrate that forms part of another embodiment of apparatus;
Fig. 3 is cross-sectional view of another embodiment of apparatus that includes the substrate shown in Fig. 2;
Fig. 4 is a schematic diagram of another embodiment where the apparatus of Fig. 3 is present in a housing.

### DEFINITIONS

Before proceeding further with a description of the specific embodiments of the present invention, a number of terms will be defined.

Polynucleotide -- a compound or composition that is a polymeric nucleotide or nucleic acid polymer. The polynucleotide may be a natural compound or a synthetic compound. In the context of an assay, the polynucleotide is often referred to as a polynucleotide analyte. The polynucleotide can have from about 2 to 5,000,000 or more nucleotides. The larger polynucleotides are generally found in the natural state. In an isolated state the polynucleotide can have about 2 to 50,000 or more nucleotides, usually about 10 to 20,000 nucleotides, more frequently 100 to 10,000 nucleotides. It is thus obvious that isolation of a polynucleotide from the natural state often results in fragmentation. The polynucleotides include nucleic acids, and fragments thereof, from any source in purified or unpurified form including DNA (dsDNA and ssDNA) and RNA, including tRNA, mRNA, rRNA, t-RNA, mitochondrial DNA and RNA, chloroplast DNA and RNA, DNA/RNA hybrids, or mixtures thereof, genes, chromosomes, plasmids, the genomes of biological material such as microorganisms, e.g., bacteria, yeasts, viruses, viroids, molds, fungi, plants, animals, humans, and the like. The polynucleotide can be only a minor fraction of a complex mixture such as a biological sample. Also included are genes, such as hemoglobin gene for sickle-cell anemia, cystic fibrosis gene, oncogenes, cDNA, and the like.

The polynucleotide can be obtained from various biological materials by procedures well known in the art. The polynucleotide, where appropriate, may be cleaved to obtain a fragment that contains a target nucleotide sequence, for example, by shearing or by treatment with a restriction endonuclease or other site specific chemical cleavage method.

The polynucleotide, or a cleaved fragment obtained from the polynucleotide, will usually be at least partially denatured or single stranded or treated to render it denatured or single stranded. Such treatments are well known in the art and include, for instance, heat or alkali treatment, or enzymatic digestion of one strand. For example, dsDNA can be heated at 90 to 100° C. for a period of about 1 to 10 minutes to produce denatured material.

Target nucleotide sequence -- a sequence of nucleotides to be identified, usually existing within a portion or all of a polynucleotide, usually a polynucleotide analyte. The identity of the target nucleotide sequence generally is known to an extent sufficient to allow preparation of various sequences hybridizable with the target nucleotide sequence and of oligonucleotides, such as probes and primers, and other molecules necessary for conducting methods in accordance with the present invention, an amplification of the target polynucleotide, and so forth.

The target sequence usually contains from about 30 to 5,000 or more nucleotides, preferably 50 to 1,000 nucleotides. The target nucleotide sequence is generally a fraction of a larger molecule or it may be substantially the entire molecule such as a polynucleotide as described above. The minimum number of nucleotides in the target nucleotide sequence is selected to assure that the presence of a target polynucleotide in a sample is a specific indicator of the presence of polynucleotide in a sample. The maximum number of nucleotides in the target nucleotide sequence is normally governed by several factors: the length of the polynucleotide from which it is derived, the tendency of such polynucleotide to be broken by shearing or other processes during isolation, the efficiency of any procedures required to prepare the sample for analysis (e.g. transcription of a DNA template into RNA) and the efficiency of detection and/or amplification of the target nucleotide sequence, where appropriate.

Oligonucleotide -- a polynucleotide, usually single stranded, usually a synthetic polynucleotide but may be a naturally occurring polynucleotide. The oligonucleotide(s) are usually comprised of a sequence of at least 5 nucleotides, preferably, 10 to 100 nucleotides, more preferably, 20 to 50 nucleotides, and usually 10 to 30 nucleotides, more preferably, 15 to 30 nucleotides.

Oligonucleotide probe -- an oligonucleotide employed to bind to a portion of a polynucleotide such as another oligonucleotide or a target nucleotide sequence. The design and preparation of the oligonucleotide probes are generally dependent upon the sensitivity and specificity required, the sequence of the target polynucleotide and, in certain cases, the biological significance of certain portions of the target polynucleotide sequence.

DNA -- deoxyribonucleic acid.

RNA -- ribonucleic acid.

cDNA -- a DNA copy of a corresponding RNA. It can be a sequence of DNA obtained by reverse transcription of an RNA molecule. It can include double-stranded or single stranded DNA obtained by amplification. An example, by way of illustration and not limitation, is the double-stranded DNA product obtained by PCR amplification of a bacterial plasmid insert. The DNA sequence inserted in the plasmid is previously obtained from reverse transcription of the corresponding RNA.

Nucleotide -- a base-sugar-phosphate combination that is the monomeric unit of nucleic acid polymers, i.e., DNA and RNA. In general, the term refers to any compound containing a cyclic furanoside-type sugar (β-D-ribose in RNA and β-D-2'-deoxyribose in DNA), which is phosphorylated at the 5' position and has either a purine or pyrimidine-type base attached at the C-1' sugar position via a β-glycosol C1'-N linkage. The nucleotide may be natural or synthetic. The term "nucleotide" as used herein includes modified nucleotides as defined below.

Nucleoside -- is a base-sugar combination or a nucleotide lacking a phosphate moiety.

Modified nucleotide -- a unit in a nucleic acid polymer that contains a modified base, sugar or phosphate group. Modified nucleotides include functional analogs (whether synthetic or naturally occurring) of such sub-units which in the polymer form (as a polynucleotide) can hybridize with naturally occurring polynucleotides in a sequence specific manner analogous to that of two naturally occurring polynucleotides. For example, these include the sub-units of PNA and other polynucleotides as described in U.S. Patent No. 5,948,902 and references cited therein (all of which are incorporated herein by reference), regardless of the source.

Hybridization (hybridizing) and binding -- in the context of nucleotide sequences these terms are used interchangeably herein. The ability of two nucleotide sequences to hybridize with each other is based on the degree of complementarity of the two nucleotide sequences, which in turn is based on the fraction of matched complementary nucleotide pairs. The more nucleotides in a given sequence that are complementary to another sequence, the more stringent the conditions can be for hybridization and the more specific will be the binding of the two sequences. Increased stringency is achieved by elevating the temperature, increasing the ratio of co-solvents, lowering the salt concentration, adding a surfactant, and the like.

Homologous or substantially identical polynucleotides -- in general, two polynucleotide sequences that are identical or can each hybridize to the same polynucleotide sequence are homologous. The two sequences are homologous or substantially identical where the sequences each have at least 90%, preferably 100%, of the same or analogous base sequence where thymine (T) and uracil (U) are considered the same. Thus, the ribonucleotides A, U, C and G are taken as analogous to the deoxynucleotides dA, dT, dC, and dG, respectively. Homologous sequences can both be DNA or one can be DNA and the other RNA.

Complementary -- Two sequences are complementary when the sequence of one can bind to the sequence of the other in an anti-parallel sense wherein the 3'-end of each sequence binds to the 5'-end of the other sequence and each A, T(U), G, and C of one sequence is then aligned with a T(U), A, C, and G, respectively, of the other sequence. RNA sequences can also include complementary G/U or U/G base pairs.

Label -- a member of a signal producing system. Usually the label is part of a target nucleotide sequence or an oligonucleotide probe, either being conjugated thereto or otherwise bound thereto or associated therewith. The label is capable of being detected directly or indirectly. Labels include (i) reporter molecules that can be detected directly by virtue of generating a signal, (ii) specific binding pair members that may be detected indirectly by subsequent binding to a cognate that contains a reporter molecule, (iii) oligonucleotide primers that can provide a template for amplification or ligation or (iv) a specific polynucleotide sequence or recognition sequence that can act as a ligand such as for a repressor protein, wherein in the latter two instances the oligonucleotide primer or repressor protein will have, or be capable of having, a reporter molecule. In general, any reporter molecule that is detectable can be used.

The reporter molecule can be isotopic or nonisotopic, usually non-isotopic, and can be a catalyst, such as an enzyme, a polynucleotide coding for a catalyst, promoter, dye, fluorescent molecule, chemiluminescent molecule, coenzyme, enzyme substrate, radioactive group, a small organic molecule, amplifiable polynucleotide sequence, a particle such as latex or carbon particle, metal sol, crystallite, liposome, cell, etc., which may or may not be further labeled with a dye, catalyst or other detectable group, and the like. The reporter molecule can be a fluorescent group such as fluorescein, a chemiluminescent group such as luminol, a terbium chelator such as N-(hydroxyethyl) ethylenediaminetriacetic acid that is capable of detection by delayed fluorescence, and the like.

Signal Producing System -- the signal producing system may have one or more components, at least one component being the label. The signal producing system generates a signal that relates to the presence or amount of a target polynucleotide in a medium. The signal producing system includes all of the reagents required to produce a measurable signal.

In their broadest application the described embodiments are directed to methods and apparatus for carrying out multiple chemical reactions. They are described for purposes of illustration primarily with regard to the hybridization of polynucleotides to surfaces such as to a surface comprising an array of polynucleotides such as oligonucleotides. However, the invention has application to other chemical reactions such as the synthesis of polynucleotides and other molecules, amplification reactions requiring the variation of temperature such as thermal cycling, and so forth. The types of chemical reactions that may be carried out using the teachings herein include, by way of illustration and not limitation, synthesis of polymeric materials such as biomolecules or biopolymers, e.g., oligonucleotides and peptides, polyalcohols such as polysaccharides, e.g., carbohydrates, oligosaccharides, plastics (polyamides, polyurethanes, polyesters, polysiloxanes) and the like; conjugation of molecules such as the conjugation of reporter groups of labels to nucleic acids or nucleotides, proteins such as enzymes, antibodies, and the like; diagnostic procedures such as those involving antibody-antigen or antibody-hapten binding, nucleic acid hybridization, and so forth; molecular biological reactions such as those involving enzymes, e.g., amplification procedures such as polymerase chain reaction, ligase chain reaction, restriction enzyme reactions; and so forth.

Any chemical reaction that may be conducted thermally, i.e., that which involves raising and lowering temperature, can be conducted in accordance with the teachings herein. For example, certain reactions may be controlled by heats of activation. When the reagents are present at the site of reaction, the temperature can be raised in accordance with the present invention to promote reaction of the reagents at the predetermined site. Other sites will not be at the proper temperature for reaction and the reagents will remain unreacted. Usually, the reagents will be globally reactive with all sites. Raising the temperature at various predetermined sites may then be used to effect reaction at such sites.

In PCR amplification temperature cycling is employed to hybridize oligonucleotide primers, extend the hybridized primers and denature the hybridized primers. The cycling is repeated a number of times to achieve a sufficient level of amplification of the target molecule. The preferred methods and apparatus may be employed to conduct PCR amplification at discrete locations on a solid substrate. Using the heat generating elements of the apparatus of the invention, temperature cycling at discrete locations can be carried by activating the heat generating elements to achieve a temperature at predetermined areas for hybridizing primers at those areas on the substrate. Then, activating the heat generating elements selectively, the temperature may be raised to a level sufficient for extension of the primers hybridized at the predetermined areas on the substrate. Next, the heat generating elements may be deactivated selectively to allow the temperature at the predetermined areas to decrease to a desired level. The cycling may be repeated at the predetermined areas to achieve the desired level of amplification.

The preferred methods may be carried out using an apparatus or device, which is brought into proximity with a reaction medium. The apparatus comprises a substrate, a plurality of discrete heat generating elements integral with the substrate and arranged in a predetermined pattern. The substrate is usually constructed from any material that is compatible with the fluids with which a surface of the substrate comes into contact. Usually, the substrate is composed of a porous or non-porous water insoluble material. The substrate can have any one of a number of shapes, such as a circle, square, rectangle, triangle, strip, plate, disk, rod, particle, including bead, and the like. In a preferred embodiment the substrate is substantially planar. The support can be hydrophilic or capable of being rendered hydrophilic and includes inorganic materials such as glass, silica, fused silica, magnesium sulfate, and alumina; natural polymeric materials, synthetic or modified naturally occurring polymers, such as poly (vinyl chloride), polyacrylamide, polyacrylate, polyethylene, polypropylene, poly(4-methylbutene), polystyrene, polymethacrylate, poly(ethylene terephthalate), nylon, poly(vinyl butyrate), etc.; either used by themselves or in conjunction with other materials; glass available as Bioglass, ceramics, metals, and the like. The substrate may be rigid or flexible. The substrate may be, for example, in the form of a semiconductor substrate or printed circuit board constructed from silicon or glass.

The heat generating elements may be any heat source that is capable of providing heat usually in response to an electrical impulse. and so forth. The heat generating elements may be, for example, resistors, electrodes, inductors, transistors or diodes and the like.

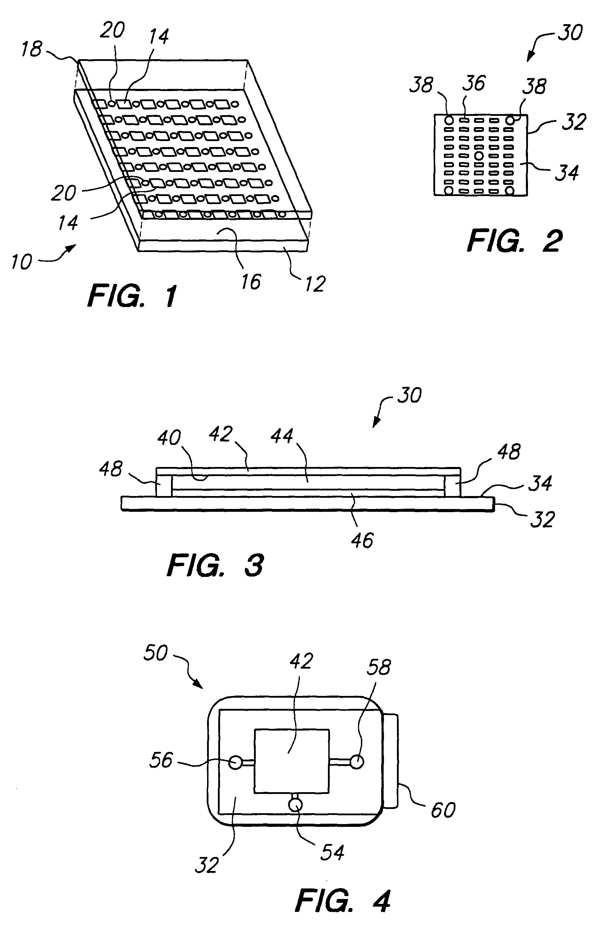

A preferred embodiment of device 10 depicted in Fig. 1. A substrate 12 has a plurality of heat generating elements 14 in the form of resistors on surface 16. Substrate 12 is substantially planar and has a length of about 1 to about 100 mm, a width of about 1 to about 100 mm and a thickness of about 0.1 to about 5 mm. The heat generating elements are arranged in any convenient pattern, which may be linear, circular, rectangular, and so forth. The heat generating elements are connected to a controller through appropriate electrical connections such as conventional wiring, conventional inter-layer metallic "vias," thin film aluminum doped with a small percentage of copper conductor film, and the like. In one approach, the heating element can be constructed simply by routing a metal trace of known width and height in a serpentine pattern. In another approach resistors can be constructed by narrowing a metal trace to a known width and height to create localized heat-generating elements. Preferably, the heating elements will be made of an aluminum alloy such as, e.g., tantalum-aluminum (TaAl) film. Metallization layers used for electrical connection may be gold, aluminum, tin, platinum, palladium, copper, and various metal combinations. In some systems, it may be desirable that the surface of the heat generating elements be flat. In this circumstance, circuitry for electrical connections may be fabricated so as to lie between the elements rather than underlying them.

As mentioned above, the heat generating elements may be activated and deactivated selectively by the use of a controller. The controller may be operated manually or preferably automatically to provide electrical impulses to the heat generating elements. The controller is usually a microprocessor combined with drive transistors to interface the heating element, a series of precise waveform generators, and the like. The apparatus may be under computer control using appropriate software to direct the activation and deactivation of the heat generating elements.

Referring to Fig. 1, device 10 may further comprise an insulating layer 18 that overlays surface 16 of substrate 12. The insulating layer is usually of a thickness approximately about 0.1 to about 100 micrometre and is made of an insulating material, i.e., a non-conductive material such as silicon dioxide and the like. The insulating layer is affixed to surface 16 by means of deposition techniques, adhesives chemical vapor deposition (CVD) and the like. Such methods are well-known in the art.

The above device may be employed to carry out chemical reactions. In one embodiment the surface of the device has bound thereto a reagent for carrying out a chemical reaction. For example, when used for polynucleotide analysis, the surface may comprise an array of polynucleotides such as oligonucleotide probes, which are bound to the surface. The reagents may be attached to the surface by conventional means. The attachment may be covalent or non-covalent. By way of illustration and not limitation, binding of oligonucleotides to a support or surface may be accomplished by well-known techniques, commonly available in the literature. See, for example, A. C. Pease, et al., Proc. Nat. Acad. Sci. USA, 91:5022-5026 (1994).

In the preferred method the device is brought into proximity with a reaction medium. In one preferred embodiment this may be accomplished by immersing the surface of the substrate with the reaction medium. Immersing may be achieved by placing the device in a container such as a singular well, microtiter plate well and the like that contains the medium. Alternatively, immersing of the surface of the substrate may be achieved by placing the medium on the surface either with or without boundaries to contain the liquid. For example, the medium may adhere to the surface by surface tension or other attractive forces. On the other hand each predetermined site on the substrate can be a well defined by walls. See, for example, Brennan, U.S. Patent No. 5,474,796, the relevant portions of which are incorporated herein by reference.

In the embodiment where the surface is immersed with the medium, there is no need for nucleation bubbles to move liquid. It is a particular advantage of this embodiment of the invention that flow cells, chambers, thin liquid films, nucleation bubbles and the like used in conducting hybridization studies and other chemical reactions in the art may be avoided.

The reaction medium may be liquid or gaseous. Usually, the reaction medium is an aqueous medium that may be polar or non-polar. The liquid may be an aqueous medium that is solely water or solely an organic solvent. An aqueous medium may contain from about 0.01 to about 97 or more volume percent of a cosolvent such as an organic solvent. Organic solvents include by way of illustration and not limitation oxygenated organic solvents of from 1-6, more usually from 1-4, carbon atoms, including alcohols such as methanol, ethanol, propanol, etc., ethers such as tetrahydrofuran, ethyl ether, propyl ether, etc., dimethylformamide, dimethylsulfoxide, 1,4-dioxane, N-methyl-2-pyrrolidone (NMP), acetonitrile and the like. Usually these cosolvents, if used, are present in less than about 70 weight percent, more usually in less than about 30 weight percent.

The pH for the liquid depends on the nature of the reagents, i.e., reagents for hybridization reactions, chemical synthesis, polynucleotide amplification, deprotection, protection, wash solution and the like and is generally selected to achieve optimum reaction between the molecules and reagents. The pH is usually in the range of about 3 to about 11, usually, about 5 to about 9. Among the factors that must be considered are the pH dependence of the reactive molecules, the stability of the molecules at different pH values, and so forth. Various buffers may be used to achieve the desired pH and maintain the pH during the reaction and thereby achieve a buffered medium. Illustrative buffers include acetate, borate, phosphate, carbonate, and the like. The particular buffer employed is not critical to this invention as long as the buffer does not react unintentionally with the reagents. Further, in an individual reaction or a wash step, one or another buffer may be preferred. For example, the buffer for hybridization reactions and wash steps may comprise sodium chloride, sodium phosphate, EDTA, lithium chloride and so forth at a pH of about 6 to about 7, usually about 7.5. For wash steps in hybridization reactions the buffered medium may also comprise a surfactant such as Triton X-100®, Tween 20® and so forth.

After the surface of the substrate is brought into proximity with the reaction medium, the heat generating elements may be selectively activated to control temperature adjacent predetermined areas on the substrate thereby controlling the chemical reactions at the predetermined areas. For example, for polynucleotide hybridization reactions the surface is brought into proximity with the reaction medium comprising the reagents for carrying out polynucleotide hybridization reactions. Typically, such reagents comprise polynucleotides, which may be, e.g., target polynucleotides or oligonucleotides, which may or may not be labeled. The medium is generally a buffered medium. The surface of the substrate usually comprises polynucleotides attached thereto in a predetermined pattern. The attached polynucleotides may be, e.g., target polynucleotides or oligonucleotides. For polynucleotide hybridization the heat generating elements are activated selectively to elevate the temperature of the medium adjacent predetermined areas on the surface to a temperature at which polynucleotide hybridization occurs. This temperature will vary depending on the nature of the polynucleotides attached to the surface and/or present in the reaction medium. In general, the temperature at the predetermined areas is usually raised to about 37 °C to about 95 °C. At this temperature some of the polynucleotides become hybridized and some of the polynucleotides are unhybridized. The substrate remains immersed in the medium for an incubation period of about 10 minutes to about 24 hours or more. Accordingly, polynucleotides hybridize to polynucleotides present at the predetermined sites leaving unhybridized polynucleotides in the reaction medium.

The apparatus may comprise a plurality of discrete temperature sensing elements integral with the substrate. In this way the temperature may be monitored in the medium adjacent predetermined areas on the substrate. The temperature sensing elements may be, for example, thermistors, thermocouples, resistive temperature device (RTD) or semiconductor temperature sensors made of resistors, diodes and monolithic IC's and the like. In Fig. 1 the substrate comprises a plurality of RTD's 20 adjacent to the heating elements 14. RTD's may be placed on or off the substrate and thermistors may be placed off the substrate in most instances. Usually, when such elements are placed off the substrate, they are conveniently on the back wall of the device. The RTD's are connected to a controller and a read-out device by appropriate electrical connections such as mentioned above for the heat generating elements. Thus, for example, in the aforementioned hybridization reactions the temperature sensing elements may be used to observe the temperature at the predetermined areas where the heat generating elements were selectively deactivated.

After the hybridization reactions the apparatus may be removed from the proximity of the medium. Thus, for example, the apparatus may be removed from the container in which it was placed and then washed. The substrate on the other hand may be separated from the reaction medium by removal of the reaction medium from the container. Alternatively, where the reaction medium was placed on the surface of the substrate, the surface may be washed to remove unreacted reagents. After separation of the reaction medium and the substrate, the temperature on the substrate may be adjusted at this time. For temperature adjustment the heat generating elements are selectively deactivated to permit the temperature at the predetermined areas to decrease. Deactivation of the heat generating elements permits the temperature at the predetermined areas to decrease to about 20 °C to about 30 °C, usually, about ambient or room temperature. The substrate may then be washed by placing in a washing solution, which may be an aqueous buffered medium as described above. If the temperature was not adjusted as discussed above prior to the immersion in the wash solution, the temperature may be adjusted after removal of the substrate from the wash solution.

The apparatus then may be analyzed to determine which polynucleotides became hybridized to the surface. In this regard the polynucleotides in the reaction medium may be labeled with a detectable label. In this approach the surface is examined for the presence of labels where the presence of labels is related to the identity of the polynucleotides and the amount of the labels is related to the amount of the polynucleotides. The nature of the labels and the material carrying the labels is described in the Definitions section above.

The results from the analysis involving exposing the substrate to the sample may optionally be processed. In this regard the results obtained from the aforementioned examining of the substrate may be processed by, for example, computer aided data analysis. In addition, the results may be forwarded to a remote location. By the term "remote location" is meant a location that is physically different than that at which the results are obtained. Accordingly, the results may be sent to a different room, a different building, a different part of city, a different city, and so forth. Usually, the remote location is at least about one mile, usually, at least ten miles, more usually about a hundred miles, or more from the location at which the results are obtained. The method may further comprise transmitting data representing the results. The data may be transmitted by standard means such as, e.g., facsimile, mail, overnight delivery, e-mail, voice mail, and the like.

In one embodiment the apparatus can comprise a plurality of electrodes integral with the substrate and arranged in a predetermined pattern. In the preferred method the electrodes may be selectively activated to control movement of reagents to and from predetermined areas on the substrate. Conveniently, the electrodes may be the heat generating elements of the device. Accordingly, in Fig. 1 the substrate comprises a plurality of heat generating elements 14, which are electrodes. The electrodes are connected to a controller by appropriate electrical connections such as mentioned above for the heat generating elements. Fixed electrodes may be plated over by processes well-known in the art of integrated circuitry with a variety of metals, including gold and nickel, chosen to be the most compatible with the attachment chemistry and the like.

In the aforementioned hybridization reactions the electrodes may be employed to move polynucleotides to and away from predetermined areas on the substrate. After the hybridization reactions have been allowed to occur, the electrodes may be activated to move unhybridized polynucleotides away from the surface. Subsequently, the substrate may be removed from proximity with the medium. In this aspect of the present invention the reagents employed are those whose movement to and away from a point of reaction may be controlled by application of electric fields and, thus, are electrically responsive. The reagent may be electrically responsive by virtue of having a charge either inherently or by virtue of introduction into the reagent of a group that creates a charge or a polarity, either positive or negative, in the reagent.

The integration of movement and temperature control in the above devices enables the system to have architecture that negates the need for flow cells, mixing means and injection or removal pumps. The aforementioned device is easy to use and requires less steps and peripheral hardware. In one embodiment the apparatus comprises an array of oligonucleotide probes on a glass slide with integrated heat generating elements and electrodes on the surface. There are two main sections on the slide, namely, the electrode, heat generating element and polynucleotide array section and the electrical interface and robot handling interface section.

The electrodes, heat generating elements and fiducials (reference or optical targets) are formed in parallel on a substrate utilizing thin film processes similar to those employed in the manufacture of an inkjet head. See, for example, "Development of the Thin-Film Structure for the ThinkJet ® Printhead," Hewlett-Packard Journal, May 1985; "The Second-Generation Thermal Inkjet Structure," Hewlett-Packard Journal, August 1988; and "The Third-Generation HP Thermal Inkjet Printhead," Hewlett-Packard Journal, August 1994, the relevant disclosures of which are incorporated herein by reference. Briefly, for example, this process involves first sputtering silicon dioxide as a barrier film. Then, the tantalum-aluminum resistor (heater) film is sputtered by magnetron. Aluminum doped with a small percentage of copper is sputtered to form the conductor film. Both of these processes are photolithographically patterned and can be used to manufacture the fiducials. A polyimide film coating further protects the passivation from the solution. The resistors (heaters) and electrical connections can be coated with a gold film to improve contact reliability and prevent corrosion.

Next, a surface compatible with polynucleotide binding is placed over the slide. Examples of such surfaces include, by way of illustration and not limitation, polylysine and the like. The polynucleotide is then deposited on the surface and final processing is completed.

A device can be designed to have as few as two sites or as many as hundreds of thousands or millions of sites. The sites may be of any shape, preferably, square or rectangular for maximizing their area. The size of a site can be varied and can be of any size, usually in the range from about 2 square micrometres to about 2 square millimeters, preferably, in the range of about 5 to about 500 square micrometres. Preferably, for hybridization reactions the substrate comprises an array of polynucleotides. In this embodiment the surface of the substrate can comprise from about 10² to about 10⁸ different polynucleotides are attached, each in an area of from about 2 micrometre by 2 micrometre to about 500 micrometre by 500 micrometre. The spacing between sites on the device is determined by the ease of fabrication, the requirement for resolution between the various sites, and the number of sites desired on a device. However, particular spacing between sites or special arrangement or geometry of the sites is not necessary for device function. Any combination of micro-locations can operate over the complete device area. Nor is it necessary to enclose the device except for an insulating or passivation layer.

A preferred procedure for utilizing the aforementioned array is described next. First, the array substrate is picked up by a robotic handler, pulse warmed to the desired temperature and then placed in a container such as a vial holding the sample to be tested. The electrodes are then activated and the sample polynucleotides are pulled towards the array. No flow cell, sample injection or mixing pumps are needed because the movement of the polynucleotides is electrically controlled. In addition, because the polynucleotides are driven towards the features, small sample volumes required in prior art methods are not necessary with the system. A high sample concentration at the surface may be achieved using the immersing technique discussed above. In addition, sample concentration may be dynamically controlled. Depending upon the hybridization time, individual polynucleotides are either hybridized on the spot or the immersed surface comprising the probe array is left in a hybridization buffer area for an allotted amount of time. During this time, mixing (polynucleotide movement) can be accomplished by alternately activating different electrodes throughout the array. In addition, because the heating of the array is accomplished by integrated heat generating elements, polynucleotides at different areas of the array can be hybridized at different temperatures or at different profiles. After hybridization, the electrodes are reversed activated to drive away nonspecific or unhybridized polynucleotides from the surface and the substrate is removed from the medium.

Two more advantages of the aforementioned apparatus are apparent. First, with the use of the apparatus and method, only polynucleotides necessary for hybridization to the features of the array are used. The remainder of the sample polynucleotides is left in the medium in the reaction container. Second, all excess sample polynucleotides can be driven away from the surface electrically before the wash step of the process is carried out. This reduces the potential for non-specific binding. Washing is accomplished by placing the apparatus in the appropriate wash solution and controlling the temperature and polynucleotide movement utilizing the same techniques described in hybridization. Again, the advantages of this system are dynamic temperature control, area specific temperature control and polynucleotide movement to and from the surface using the electrodes. After washing, the array can be scanned on a polynucleotide scanner. Finally, the polynucleotides on the substrate array can be recovered by placing the substrate back into a medium, activating the heat generating elements to dehybridize the sample polynucleotides and finally driving the polynucleotides away from the array by activating the electrodes. Alternatively, the substrate array can be discarded if no sample recovery is needed.

It is, however, within the scope of the teachings herein to use the aforementioned principles in conjunction with certain features of the prior art such as chambers into which thin films of fluid are placed. The present invention may also be used in conjunction with nucleation bubbles to move fluid within the chamber.

In an alternative embodiment, an apparatus comprises first and second substrates. Referring to Fig. 2, an apparatus 30 is shown comprising a first substrate 32 having a surface 34. The substrate may be composed of any material that is compatible with the fluids with which the surface comes in contact with such as set forth above. The surface area of the inner surface that comes in contact with the fluid may be on the order of about 10 mm² to about 20 cm², more preferably from about 100 mm² to about 10 cm², and most preferably about 200 mm² to about 10 cm². The volume of the fluid that can be retained by surface 34 is less than about 300 µl, more preferably less than about 150 µl, even more preferably less than about 50 µl.

Surface 34 of first substrate 32 has a plurality of resistors 36, which act as discrete heat sources. The resistors are adjacent to the inner surface of the substrate and in thermal communication with the film of fluid retained thereon. The resistors are electrically connected to a control circuit that selectively controls the voltages or currents applied to the resistors. Substrate 32 also comprises a plurality of temperature sensing elements 38. In apparatus 30 substrate 32 is positioned such that surface 34 is opposite surface 40 of second substrate 42. Surface 40 has a plurality of polynucleotides attached thereto. The area between the substantially parallel surface 34 and surface 40 defines a fluid chamber 44 (see Fig. 3). Chamber 44 may be formed as a closed chamber by employing a seal 48 attached to the outer periphery of the substantially parallel substrates. The closed chamber may be a micrometre to several millimeters in thickness, preferably from about 5 micrometres to about 100 micrometres in thickness. Fluid in chamber 44 is protected from surface 34 of substrate 32 by an insulating or passivation layer 46.

Seal 48 between the two opposing inner surfaces can be solid or flexible, and is fabricated from, for example, adhesives, rubber, plastic, glass, or metal. The apparatus may preferably include an opening in one of the substrates or in the seal for introducing fluid into the closed chamber. The opening may be a port or other entrance. The fluid may be introduced by centrifugal means, pressure means, vacuum means, positive displacement means, or other means known in the art. Fluid may be applied to an application site near the center of the apparatus, and then distributed across the first surface to give the film of fluid. The fluid can be distributed across the inner surfaces of the substrate by, for example, rotating the apparatus and the like.

Surface 34 may comprise the heat generating elements and the temperature sensing elements as well as the attached polynucleotides. Alternatively, surface 34 may have the heat generating elements and the temperature sensing elements and surface 40 may comprise the attached polynucleotides. Alternatively, both surfaces 34 and 40 may comprise the heat generating elements and the temperature sensing elements as well as the attached polynucleotides.

The aforementioned assembly may be placed in a suitable housing for handling such as depicted in Fig. 4. Housing 50 may be fabricated from plastic, glass, silicon, ceramic and the like. The surface 52 of housing 50 may comprise port 54 for loading sample into chamber 44 of housing 50. Surface 52 may also comprise second port 56 for loading wash buffer into chamber 44. Also, surface 52 may comprise third port 58 used to permit air or other gases to exhaust from chamber 44. Referring to Fig. 4, electrical connections 60 are shown for making electrical connections to resistors 36 and to temperature sensors 38.

As mentioned above, the preferred apparatus may be employed in conjunction with certain features known in the art. For example, the heat generating elements may be used to selectively heat the medium adjacent predetermined areas on the substrate as well as to nucleate bubbles to provide for mixing as described in Schembri, *supra.* During the chemical reactions using the present apparatus and method, the heat generating elements are not activated to the level necessary to raise the temperature of the medium to permit nucleation of bubbles.

If bubble nucleation is employed in the present methods for mixing of fluids, bubbles are nucleated by heating the fluid in the local environment around the heat generating elements. Subsequently, the control circuit may selectively reduce the electrical input to the heat generating elements to allow the bubble to dissipate or collapse thereby mixing the fluid. The fluid can be mixed locally by moving a bubble back and forth between two neighboring heat sources. Alternatively, a plurality of bubbles can be nucleated at a plurality of locations and moved independently of one another.

It is to be understood that while specific embodiments have been described, the foregoing description as well as the examples that follow, are intended to illustrate and not limit the scope of the invention. Other aspects, advantages and modifications within the scope of the claims will be apparent to those skilled in the art.

### EXAMPLES

### Example 1

### Hybridization to DNA arrays

An array of DNA probes is constructed by attaching a plurality of known probes comprising oligomers, PCR product, or cDNA at specific locations on the inner surface of the substrate using techniques well known in the art. The substrate is a rectangular glass slide having a surface area of about 100 mm², and having resistors on the inner surface that are connected to a electrical source. The resistors and RTD's are formed on the substrate by photolithographical thin film processes as described earlier.

The procedure for creating the attachment chemistry is sometimes referred to a "priming" the surface. To this end, the top surface of the substrate is modified so as to prepare the surface for attachment of the appropriate material. The surface may be modified with groups or coupling agents to covalently link the desired molecules to the surface at the predetermined sites. Representative groups include, by way of illustration and not limitation, amino, especially primary amino, hydroxyl, thiol, sulfonic acid, phosphorous and phosphoric acid, particularly in the form of acid halides, especially chloride and bromide, and carboxyl, and the like. The reactive groups are conveniently attached to the surface commonly through a hydrocarbyl radical such as an alkylene or phenylene divalent radical. Such hydrocarbyl groups may contain up to 10 carbon atoms. One preferred procedure for the derivatization of the metal electrode surface uses and aminoalkyl silane derivative, e.g., trialkoxy 3-aminopropylsilane such as aminopropyltriethoxy silane (APS), 4-aminobutyltrimethoxysilane, 4-aminobutyltriethoxysilane, 2-aminoethyltriethoxysilane, and the like. APS reacts readily with the oxide and/or hydroxyl groups on metal and silicon surfaces. APS provides primary amine groups for the subsequent covalent coupling reactions of the attachment of oligonucleotides and other molecules. Such a procedure is described in EP-B-0,173,356, the relevant portions of which are incorporated herein by reference. While this represents one of the preferred approaches, a variety of other attachment reactions are possible for both the covalent and non-covalent attachment as mentioned above.

The substrate comprising the array is placed in a well containing about 40µl of reaction medium comprising a sample of DNA in a buffer (900 mM sodium chloride/ 60 mM sodium phosphate/ 6 mM EDTA, pH7.5). The resistors on the substrate are selectively activated to raise the temperature of the medium adjacent predetermined areas on the substrate to 37°C. The period of incubation at this temperature is 1 hour. Temperature at the predetermined areas of the substrate is monitored by selectively activating the thermistors.

After hybridization, the array is washed in the aforementioned buffer containing 0.005% w/v Triton X-100 at 37°C for 15 minutes. In this step elevation of the temperature is achieved by utilizing the selective activation of the resistors as discussed above. Alternatively, conventional means are employed to raise the temperature of the bulk medium. The array is rinsed and then dried. The array is scanned dry for the presence and amount of label, which in this example is a fluorescent label conveniently fluorescein. The array images are quantitated by integrating the measured intensity over the substrate centered on each feature.

For surface probes that are cDNA the resistors are selectively activated to heat the medium adjacent predetermined areas on the substrate to 65°C for hybridization studies where the surface probes are cDNA.

All publications and patent applications cited in this specification are herein incorporated by reference as if each individual publication or patent application were specifically and individually indicated to be incorporated by reference.

The disclosures in United States patent application no. 09/626,419, from which this application claims priority, and in the abstract accompanying this application are incorporated herein by reference.

## Claims

1. A method of carrying out hybridization reactions involving biopolymers, including the steps of:
(a) bringing into proximity with a reaction medium a substrate comprising a plurality of heat generating elements, said reaction medium comprising reagents for carrying out said hybridization reactions, and
(b) selectively activating and deactivating said heat generating elements to control temperature of said medium adjacent predetermined areas on said substrate thereby controlling said hybridization reactions adjacent said predetermined areas.

2. A method according to claim 1, wherein a surface of said substrate comprises a plurality of biopolymers attached thereto in a predetermined pattern.

3. A method according to claim 1 or 2, wherein said biopolymers are polynucleotides.

4. A method of carrying out polynucleotide hybridization reactions, including the steps of:
(a) bringing into proximity with a reaction medium a substrate comprising a plurality of heat generating elements, said reaction medium comprising reagents for carrying out said reactions, wherein a surface of said substrate comprises a plurality of polynucleotides bound thereto in a predetermined pattern; and
(b) selectively activating and deactivating said heat generating elements to control temperature of said medium adjacent predetermined areas on said substrate thereby controlling said hybridization reactions adjacent said predetermined areas.

5. A method according to any preceding claim, wherein said substrate comprises a plurality of temperature sensing elements and said method comprises selectively activating said temperature sensing elements to monitor said temperature adjacent said predetermined areas on said substrate

6. A method according to any one of claims 1 to 4, wherein said substrate comprises a plurality of electrodes and said method comprises selectively activating said electrodes to control movement of said reagents relative to said substrate.

7. A method according to any preceding claim, wherein said substrate comprises an insulating layer.

8. A method according to any preceding claim, wherein said discrete heat generating elements comprise resistors arranged in a predetermined pattern and/or discrete temperature sensing elements comprise thermistors arranged in a predetermined pattern.

9. Apparatus for conducting polynucleotide hybridization reactions, including:
a substrate,
a plurality of discrete heat generating elements arranged in a predetermined pattern on said substrate, and
a plurality of discrete temperature sensing elements on said substrate.

10. Apparatus according to claim 9, including a plurality of electrodes arranged in a predetermined pattern on said substrate.

11. Apparatus according to claim 9 or 10, wherein said substrate comprises an insulating layer.

12. Apparatus according to claim 10, 11 or 12, wherein said discrete heat generating elements comprise resistors arranged in a predetermined pattern and said discrete temperature sensing elements comprise thermistors arranged in a predetermined pattern.

13. Apparatus according to any one of claims 9 to 12, wherein a surface of said substrate comprises polynucleotides attached thereto in a predetermined pattern.

14. Apparatus according to claim 13, wherein from about 10² to about 10⁸ different polynucleotides are attached, each in an area of from about 2 micrometre by 2 micrometre to about 500 by 500 micrometre.

15. A method of carrying out polynucleotide hybridization reactions, including the steps of:
(a) bringing into proximity with a reaction medium a substrate comprising a plurality of resistors, wherein a surface of said substrate comprises an array of polynucleotides attached thereto, said reaction medium comprising reagents for carrying out said polynucleotide hybridization reactions,
(b) selectively activating and deactivating said heat generating elements to control temperature of said medium adjacent predetermined areas on said substrate to control hybridization of polynucleotides at said predetermined areas thereby forming hybridized polynucleotides and unhybridized polynucleotides.

16. A method of carrying out polynucleotide hybridization reactions, including the steps of:
(a) bringing into proximity with a reaction medium a substrate comprising a plurality of electrodes, a plurality of heat generating elements and a plurality of temperature sensing elements, wherein a surface of said substrate comprises a plurality of polynucleotides bound thereto in a predetermined pattern and wherein said reaction medium comprises reagents for carrying out said polynucleotide hybridization reactions,
(b) selectively activating said electrodes to control movement of said reagents to and from predetermined areas adjacent said substrate,
(c) selectively activating and deactivating said heat generating elements to control temperature of said medium adjacent predetermined areas on said substrate thereby controlling reaction of biopolymers adjacent said predetermined areas, and
(d) selectively activating said temperature sensing elements to monitor said temperature adjacent said predetermined areas on said substrate.

17. A method according to claim 16, wherein from about 10² to about 10⁸ different polynucleotides are attached to said surface, each in an area of from about 2 micrometre by 2 micrometres to about 500 by 500 micrometres.
